# EUROPÄISCHE PATENTSCHRIFT

(11) **EP 0 402 667 B1**
(45) Veröffentlichungstag und Bekanntmachung des Hinweises auf die Patenterteilung: **02.02.1994**
(21) Anmeldenummer: 90109780.8
(22) Anmeldetag: 23.05.1990
(51) Int. Cl.: C07D 233/64, C12Q 1/28, G01N 33/52, C07D 455/04

(54) **Diarylimidazole**
Diarylimidazoles
Diarylimidazoles

(30) Priorität: 31.05.1989 DE 3917677
(43) Veröffentlichungstag der Anmeldung: 19.12.1990
(73) Patentinhaber: BOEHRINGER MANNHEIM GMBH, 68298 Mannheim (DE)
(72) Erfinder: Braun, Hans-Peter, Dr. rer. nat., D-6940 Weinheim (DE); Deneke, Ulfert, Dr. rer. nat., D-6149 Rimbach-Zotzenbach (DE); Güthlein, Werner, Dr. rer. nat., D-6800 Mannheim 24 (DE); Nagel, Rolf, D-6842 Bürstadt (DE)

(56) Entgegenhaltungen:
- EP-A- 0 116 307
- EP-A- 0 122 641
- EP-A- 0 161 436
- DE-A- 2 735 690
- JOURNAL OF ORGANIC CHEMISTRY Band 17, 1952, Seiten 1281-1289; P.A.S. SMITH et al.: "Preparation and Properties of Some Substitued Julolidines"

## Beschreibung

Die Erfindung betrifft die Verwendung von Diarylimidazolen als Redoxindikatoren, insbesondere in Gegenwart von einer eine oder mehrere SH-Gruppen enthaltenden Substanz.

Ein weiterer Gegenstand der Erfindung ist ein Verfahren zur Bestimmung eines Probeninhaltsstoffes, wobei eine Probe mit einer eine oder mehrere SH-Gruppen enthaltenden Substanz versetzt und mit einem Testsystem kontaktiert wird, das mit dem in der Probe zu bestimmenden Inhaltsstoff Wasserstoffperoxid bildet, welches kolorimetrisch bestimmt wird.

Außerdem betrifft die Erfindung ein Mittel zur Bestimmung eines Probeninhaltsstoffes, das eine eine oder mehrere SH-Gruppen tragende Substanz, ein Testsystem zum Nachweis des Probeninhaltsstoffes durch Bildung von Wasserstoffperoxid und einen Redoxindikator zur Bestimmung von Wasserstoffperoxid in Gegenwart von Peroxidase enthält.

Desweiteren betrifft die Erfindung Diarylimidazole der allgemeinen Formel I 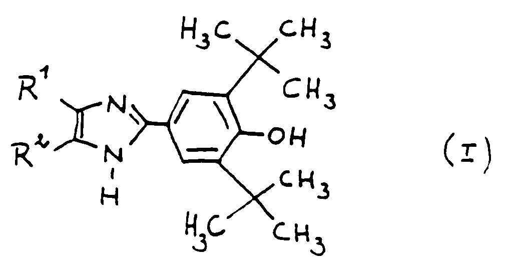
in der
R¹ einen Rest der allgemeinen Formel II 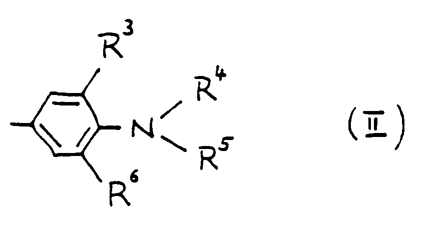
bedeutet, wobei
R³ und R⁶ Wasserstoff und
R⁴ und R⁵, die gleich oder verschieden sein können, Niederalkyl oder
R³/R⁴ oder/und R⁵/R⁶ jeweils zusammen einen Niederalkylenrest darstellen und
R² für einen Niederalkylrest steht
sowie deren Salze,
mit Ausnahme der Verbindung 2-(3′,5′-Di-tert.butyl-4′-hydroxyphenyl)-4(5)-(4˝-dimethylaminophenyl)-5(4)-methyl-(1H)-imidazolhydrochlorid sowie ein Verfahren zur Herstellung der vorgenannten Diarylimidazole.

Manche Enzyme besitzen freie SH-Gruppen. Werden diese oxidiert, nimmt die enzymatische Aktivität ab oder das Enzym wird völlig inaktiviert. Ein Beispiel für solche Enzyme ist die Creatin-Kinase. Die genaue Bestimmung der Anwesenheit und Menge an Creatin-Kinase in Körperflüssigkeiten, insbesondere in Blutserum, ist von erheblicher Bedeutung für die Prognose des Myocardinfarkts oder anderer Myopathien.

Für den speziellen Fall der Creatin-Kinase ist es bekannt, daß bei dieser durch Oxidation z. B. an der Luft, freie SH-Gruppen zu Disulphiden oxidiert werden und daß das Enzym damit einen Teil seiner Aktivität verliert. Die Reaktivierung kann durch Zugabe von Substanzen erfolgen, die eine oder mehrere freie SH-Gruppen enthalten. Als solche reaktivierend wirkenden SH-Reagenzien sind Substanzen, wie Glutathion, N-Acetylcystein, Dithioerythritol, Dithiotreitol, Mercaptoethanol oder Thioglucose bekannt. Allerdings führt die Anwendung solcher reduzierend wirkender Verbindungen in Nachweissystemen, mit denen die zu bestimmende Enzymaktivität mittels eines Redoxindikators, d. h. einer Substanz, die infolge Oxidation oder Reduktion ihre Farbe ändert, bestimmt werden soll, im allgemeinen zu Störungen und zu falschen Resultaten. Insbesondere ist dies der Fall, wenn für eine Nachweisreaktion die enzymatische Bildung von Wasserstoffperoxid und dessen Bestimmung mittels eines Redoxindikators in Gegenwart von Peroxidase ausgenutzt werden soll, was sehr häufig der Fall ist. Es hat deshalb nicht an Bemühungen gefehlt, solche Redoxindikatoren und Testsysteme zur Verfügung zu stellen, die diesbezüglich nicht störanfällig sind.

Aus EP-A-0 116 307 ist ein Mittel zur kolorimetrischen Bestimmung von Creatin-Kinase bekannt, das Triarylimidazole gemäß DE-A-27 35 690 enthält. Diese Imidazole sind jedoch nur im sauren pH-Bereich stabil und werden - wie Experimente zeigten - beim Überführen in einen schwach sauren, neutralen bis schwach alkalischen pH-Bereich, wie er bei vielen enzymatischen Reaktionen notwendig ist, sehr leicht oxidiert. Außerdem sind die beschriebenen Triarylimidazole gegenüber SH-Gruppen tragenden Substanzen so empfindlich, daß deren Konzentration 0,2 mMol/l in der zu bestimmenden Testlösung nicht überschreiten darf. Eine so niedrige Konzentration von z. B. N-Acetylcystein reicht jedoch oft nicht zur vollständigen Reaktivierung von Creatin-Kinase aus.

Als Redoxindikatoren für die Bestimmung von Wasserstoffperoxid werden in EP-A-0 122 641 Diarylimidazole empfohlen. Die genannten Substanzen zeichnen sich dadurch aus, daß die Aryl-Gruppe in Position 2 des Imidazolrings keinen Alkyl-Substituenten tragen kann und als Alkyl-Substituenten in Position 5 des Imidazolringes nur höhere Alkyl-Homologe in Frage kommen.

In EP-B-0 161 436 werden Diarylimidazole beschrieben, die im schwach sauren bis alkalischen Bereich von Luftsauerstoff nicht spontan oxidiert werden und die mit in Serum enthaltenen Substanzen nicht reagieren. Über die Eigenschaften der Diarylimidazole in Gegenwart von SH-Gruppen tragenden Substanzen, die die zu untersuchende Probe ursprünglich nicht enthält, wird nichts ausgesagt. Wie Überprüfungen gezeigt haben, sind die in den beiden europäischen Anmeldungen als vorteilhaft beschriebenen Diarylimidazole empfindlich gegenüber SH-Gruppen tragenden Substanzen, wie sie z. B. zur Reaktivierung von Creatin-Kinase gebraucht werden. Eine Bestimmung von Creatin-Kinase mittels der als vorteilhaft beschriebenen Diarylimidazole ist in Anwesenheit von Konzentrationen an SH-Gruppen tragenden Substanzen, die zur vollständigen Reaktivierung von Creatin-Kinase erforderlich sind, nicht ungestört möglich.

Die Aufgabenstellung der vorliegenden Erfindung war es deshalb, Redoxindikatoren, insbesondere für den Nachweis von Wasserstoffperoxid bzw. peroxidatisch wirksamen Substanzen, speziell für Verfahren und Mittel zur Bestimmung von Probeninhaltsstoffen, wie z. B. Creatin-Kinase zur Verfügung zu stellen, die die vorstehenden Nachteile nicht aufweisen und die insbesondere durch SH-Gruppen tragende Substanzen nicht beeinflußt werden.

Die Aufgabe wird durch die Erfindung, wie sie in den Ansprüchen gekennzeichnet ist, gelöst.

Überraschenderweise wurde gefunden, daß Diarylimidazole der allgemeinen Formel I 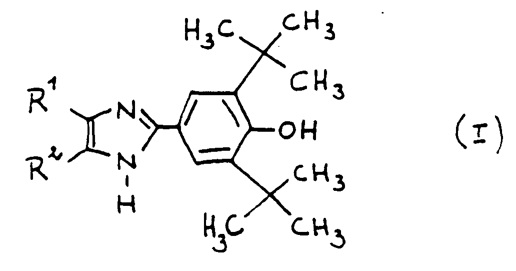
in der R¹ einen Rest der allgemeinen Formel II 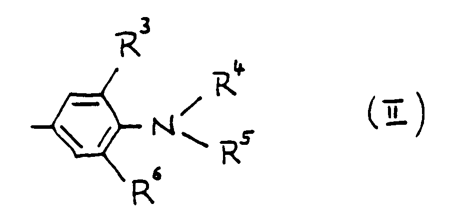
bedeutet, wobei
R³ und R⁶ Wasserstoff und
R⁴ und R⁵, die gleich oder verschieden sein können, Niederalkyl oder
R³/R⁴ oder/und R⁵/R⁶ jeweils zusammen einen Niederalkylenrest darstellen und
R² für einen Niederalkylrest steht
sowie deren Salze
den oben gestellten Anforderungen entsprechen.

Gegenstand der Erfindung ist deshalb die Verwendung eines Diarylimidazols der allgemeinen Formel I, wie vorstehend beschrieben, als Redoxindikator.

In der vorstehend genannten Formel I bedeutet Niederalkyl in der Definition von R², R⁴ und R⁵ einen 1 -6, vorzugsweise 1 - 4 Kohlenstoffatome enthaltenden Rest. Besonders bevorzugt ist die Methylgruppe.

Für den Fall, daß R³/R⁴ und R⁵/R⁶ jeweils zusammen einen Niederalkylenrest darstellen, ist hierunter eine 2 oder 3, vorzugsweise 3 Kohlenstoffatome enthaltende Gruppe zu verstehen.

Erfindungsgemäß besonders bevorzugte Diarylimidazole sind solche, bei denen R¹ einen p-Dimethylaminophenylrest, einen p-Diethylaminophenylrest, einen p-Di-n-propylaminophenylrest oder einen Julolidinrest und R² eine Methylgruppe darstellt. Ganz besonders bevorzugt ist 2-(3′,5′-Di-tert.-butyl-4′-hydroxyphenyl)-4(5)-(9˝-julolidyl)-5(4)-methyl-(1H)-imidazol.

Die Diarylimidazole der allgemeinen Formel I liegen insbesondere in Lösung mit ihrer entsprechenden tautomeren Form (I a) 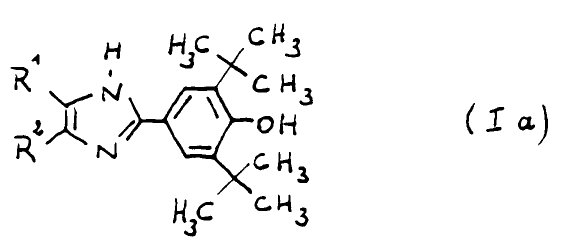
im Gleichgewicht. Die Darstellung der Formel I soll erfindungsgemäß die entsprechenden tautomeren Verbindungen mit einschließen. In der Bezeichnung des erfindungsgemäß ganz besonders bevorzugten Diarylimidazols bedeuten die Klammerausdrücke (4) bzw. (5) die jeweilige Substituentenposition der tautomeren Imidazolform.

Die erfindungsgemäß einzusetzenden Diarylimidazole können als freie Basen oder als Salze vorliegen. Es hat sich gezeigt, daß die Salze eine noch höhere Stabilität aufweisen als die freien Basen. Als Salze kommen insbesondere die Salze starker Säuren in Frage, wie Hydrochlorid, Sulphat, Tetrafluoroborat oder Salze aromatischer Sulphonsäuren, wie der para-Toluolsulphonsäure oder der Naphthalin-2-sulfonsäure. Ganz besonders bevorzugt sind die Hydrochloride der Diarylimidazole der allgemeinen Formel I.

Die erfindungsgemäß zu verwendenden Diarylimidazole der allgemeinen Formel I sind farblose bis schwach beige oder schwach rosa gefärbte Verbindungen, die nach Oxidation stark gefärbt sind.

Diarylimidazole der allgemeinen Formel I sind so stabil, daß sie im schwach sauren bis alkalischen Bereich von Luftsauerstoff nicht spontan oxidiert werden. Außerdem können mit den erfindungsgemäß zu verwendenden Verbindungen Wasserstoffperoxid bzw. peroxidatisch wirkende Substanzen auch in Gegenwart von SH-Gruppen tragenden Substanzen, wie z. B. Glutathion, N-Acetyl-cystein, Dithioerythritol, Dithiotreitol, Mercaptoethanol oder Thioglucose nachgewiesen werden. Beispielsweise können Konzentrationen bis zu etwa 25 mmol/l N-Acetylcystein diese Nachweisreaktion nicht stören. Die Diarylimidazole der allgemeinen Formel I können deshalb vorteilhaft in Gegenwart von einer eine oder mehrere SH-Gruppen enthaltenden Substanz als Redoxindikator verwendet werden. Ganz besonders vorteilhaft verwendbar sind hierzu die im Vorangehenden als bevorzugt genannten Verbindungen.

Aufgrund der vorgenannten vorteilhaften Eigenschaften sind Diarylimidazole der allgemeinen Formel I speziell für die Bestimmung der Aktivität des Enzyms Creatin-Kinase, das durch SH-Gruppen-haltige Agenzien reaktiviert wird, geeignet.

Mit den erfindungsgemäßen Verbindungen der allgemeinen Formel I, insbesondere mit den bevorzugten Diarylimidazolen ist die Durchführung eines Verfahrens zur Bestimmung eines Probeninhaltsstoffes vorteilhaft möglich, bei dem die Probe mit einer eine oder mehrere SH-Gruppen enthaltenden Substanz versetzt und mit einem Testsystem kontaktiert wird, das mit dem in der Probe zu bestimmenden Inhaltsstoff Wasserstoffperoxid bildet, welches seinerseits kolorimetrisch bestimmt wird. In einem solchen erfindungsgemäßen Verfahren wird der zu untersuchenden Probe, wie beispielsweise Körperflüssigkeiten, insbesondere Blut, Serum, Plasma und Urin, eine eine oder mehrere SH-Gruppen enthaltende Substanz zugegeben, um z. B. eventuell oxidierte Probeninhaltsstoffe, wie beispielsweise Creatin-Kinase in der Probe zu reaktivieren. Als SH-Gruppen enthaltende Substanzen können hierzu beispielsweise eingesetzt werden Glutathion, N-Acetyl-Cystein, Dithioerithritol, Dithiotreitol, Mercaptoethanol oder Thioglucose. Besonders bevorzugt ist der Einsatz von N-Acetylcystein.

Die Konzentration der für eine zuverlässige Bestimmung von Probeninhaltsstoffen gegebenenfalls notwendigen SH-Gruppen-haltigen Substanzen ist von der zu bestimmenden Substanz abhängig und ist dem Fachmann bekannt oder kann durch einfache Versuche ermittelt werden. Um z. B. eine vollständige Reaktivierung von Creatin-Kinase in einer Blutprobe zu erzielen, wird soviel einer eine oder mehrere SH-Gruppen tragenden Substanz zugesetzt, daß die Konzentration in der zu untersuchenden Probe vorzugsweise bei 2 bis 20 mmol/l liegt. Unterhalb 2 mmol/l ist in der Regel keine vollständige Enzymaktivierung möglich.

Das für die Bestimmung eines Probeninhaltsstoffes notwendige Testsystem richtet sich nach dem jeweiligen Probeninhaltsstoff. Dem Fachmann sind jeweils geeignete Testsysteme bekannt. Beispielsweise wird bei dem sich an die Reaktivierung von Creatin-Kinase anschließenden Nachweisverfahren üblicherweise von einer Kopplung enzymatischer Reaktionen Gebrauch gemacht, wobei letztendlich Wasserstoffperoxid gebildet und bestimmt wird, aus dessen Menge bzw. Umsatz pro Zeiteinheit auf die Menge an Creatin-Kinase geschlossen werden kann. Bewährt hat sich ein Testsystem, in dem aus Creatinphosphat und ADP in Anwesenheit eines zweiwertigen Metallkations durch Creatin-Kinase-Aktivität ATP und Creatin gebildet wird. ATP kann dann mit einem zweiwertigen Metallkation und Glycerokinase zur Phosphorylierung von Glycerin eingesetzt werden. Anschließende Oxidation des gebildeten Glycerin-3-phosphates mit Luftsauerstoff und Glycerin-3-phosphatoxidase führt dann zur Bildung von Wasserstoffperoxid.

Erfindungsgemäß dient das gebildete Wasserstoffperoxid für eine durch Peroxidase katalysierte Oxidation eines Diarylimidazols der allgemeinen Formel I. Hierdurch wird eine im sichtbaren Wellenlängenbereich absorbierende Farbe gebildet, welche prinzipiell für die Bestimmung jeden Probeninhaltsstoffes verwendet werden kann, dessen Nachweis über die Bildung von Wasserstoffperoxid erfolgt, speziell und besonders vorteilhaft aber zur quantitativen Bestimmung der Creatin-Kinase herangezogen werden kann. Die Anwesenheit einer eine oder mehrere SH-Gruppen enthaltenden Substanz stört hierbei nicht. Für das erfindungsgemäße Verfahren hat sich insbesondere für die Bestimmung von Creatin-Kinase aus Blut, Serum oder Plasma als besonders vorteilhaft die Kombination von N-Acetylcystein als SH-Gruppe tragende Substanz und 2-(3′,5′-Di-tert.-butyl-4′-hydroxyphenyl)-4(5)-9˝-julolidyl-5-(4)-methyl-(1H)-imidazol erwiesen.

Das erfindungsgemäße Verfahren ist in Lösung, z. B. in einer Küvette durchführbar, besonders vorteilhaft kann es aber trägergebunden durchgeführt werden.

Hierbei sind die für das erfindungsgemäße Verfahren notwendigen Reagenzien auf saugfähige oder quellfähige Materialien, die dem Fachmann als Reagenzträger bekannt sind, wie Papiere, Vliese oder Filme etc. aufgebracht. Solche Reagenzträger können als Schnelldiagnostica zur direkten Bestimmung von Probeninhaltsstoffen, wie z. B. Creatin-Kinase in Körperflüssigkeiten, wie Blut, Serum oder Plasma, eingesetzt werden. Dazu wird die Körperflüssigkeit direkt auf den Reagenzträger gebracht oder dieser in diese eingetaucht. Durch Vergleichen der entstandenen Farbe mit Vergleichsfarben ist eine semiquantitative Bestimmung möglich. Über remissionsphotometrische Verfahren, die dem Fachmann bekannt sind, kann quantitativ ausgewertet werden.

Ein erfindungsgemäßes Mittel zur Bestimmung eines Probeninhaltsstoffes, beispielsweise von Creatin-Kinase enthaltend eine eine oder mehrere SH-Gruppen tragende Substanz, ein Testsystem zum Nachweis des Probeninhaltsstoffes, beispielsweise von Creatin-Kinase durch Bildung von Wasserstoffperoxid und einen Redoxindikator zur Bestimmung von Wasserstoffperoxid in Gegenwart von Peroxidase ist dadurch gekennzeichnet, daß der Redoxindikator ein Diarylimidazol der allgemeinen Formel I ist. Ein solches Mittel kann in Lösung oder als Lyophilisat, Pulvermischung oder als Tablette verpreßt zur Verwendung in Lösung vorliegen. Besonders vorteilhaft ist das erfindungsgemäße Mittel in Form eines Testträgers. Hierbei liegen die zur Bestimmung des Probeninhaltsstoffes, beispielsweise von Creatin-Kinase erforderlichen Substanzen auf feste Materialien aufgebracht vor. Ein solcher Testträger kann saugfähige Reagenzträgermaterialien, wie Filterpapier, Vliese aus z. B. Glas oder Kunststoff oder quell- bzw. saugfähige Reagenzfilme enthalten.

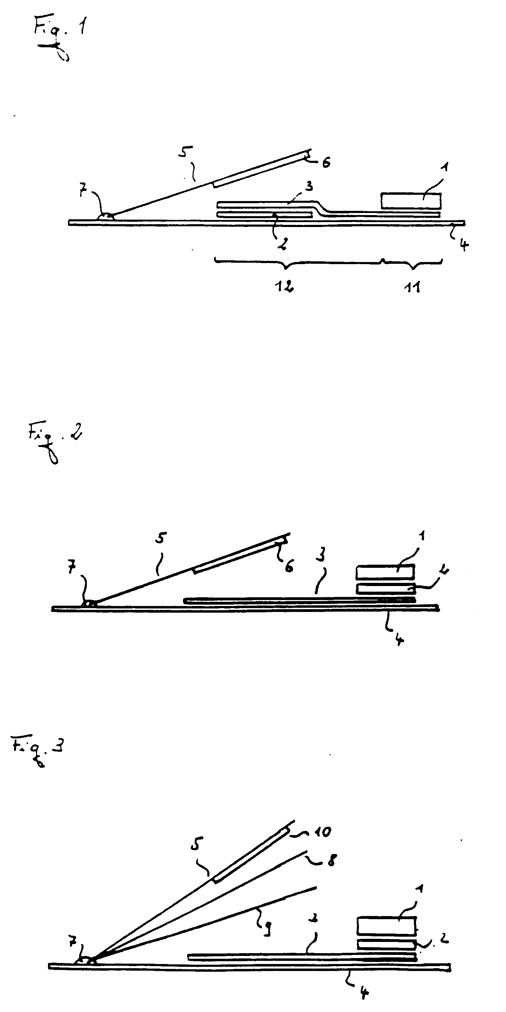

In den Figuren 1, 2 und 3 sind im Querschnitt drei Testträger dargestellt, die für die Bestimmung von Probeninhaltsstoffen, insbesondere aber für die Bestimmung von Creatin-Kinase aus Blut geeignet sind. Sie erlauben einerseits aus Vollblut Serum bzw. Plasma abzutrennen und andererseits aufgrund eines speziell gestalteten Aufbaus bzw. Anordnung der Reagenz- und Hilfsstoffschichten bzw. -träger eine Temperierung, Vorreaktion und gezieltes Starten der Hauptreaktion. Die einzelnen Testträger unterscheiden sich im wesentlichen nur durch die Anordnung des die SH-Gruppen-haltige Substanz enthaltenden Reagenzträgers und durch die Art, wie die Reagenzien des Testsystems vorliegen. Im einzelnen sind die Vorrichtungen wie folgt zusammengesetzt:

Figur 1: Auf einer inerten Trägerfolie (4), beispielsweise einer Kunststoffolie, ist eine Transportschicht (3) befestigt, die zum Transport der Probenflüssigkeit aus der Probenaufgabezone (11) in den Nachweisbereich (12) dient. Als Transportschicht ist prinzipiell jedes Material geeignet, das die zu untersuchende Flüssigkeit aus der Probenaufgabezone (11) in den Nachweisbereich (12) zu transportieren in der Lage ist und sie dabei nicht verändert. Besonders vorteilhaft ist als Transportschicht ein Glasfaservlies einzusetzen. Die Schicht (3) teilweise überdeckend ist eine Schicht zur Abtrennung von korpuskulären Bestandteilen aus der Probenflüssigkeit (1) befestigt. Grundsätzlich kann hierfür jedes Material verwendet werden, das erlaubt korpuskuläre Bestandteile aus der Probenflüssigkeit, insbesondere Blutzellen, vor allem Erythrozyten aus Blut abzutrennen und nicht in den Nachweisbereich (12) gelangen zu lassen, wo diese zu Störungen der Nachweisreaktion Anlaß geben könnten. Andererseits darf das Material für Schicht (1) die Probenflüssigkeit nicht so beeinflussen, daß die Bestimmung des gewünschten Probeninhaltsstoffes gestört oder verfälscht wird. Als besonders geeignet für Schicht (1) haben sich Glasfaservliese erwiesen, wie sie z. B. in EP-B-0 045 476 beschrieben sind. Im Nachweisbereich (12) ist zwischen Transportschicht (3) und Trägerfolie (4) die Aktivierungsschicht (2) angeordnet, die eine eine oder mehrere SH-Gruppen tragende Substanz enthalt. Die Aktivierungsschicht (2) besteht vorzugsweise aus einem dünnen saugfähigen Material, vorzugsweise aus einem multifilen Gewebe, das mit der aktivierenden Substanz imprägniert ist. Darüber hinaus kann die Aktivierungsschicht aber auch noch andere Substanzen enthalten, z. B. solche, die störende Stoffe aus der zu untersuchenden Flüssigkeit beseitigen. Beispielsweise kann Ascorbatoxidase zur Entfernung von Vitamin C in der Aktivierungsschicht mit enthalten sein. Seitlich von Transportschicht (3) ist über eine Klebestelle (7) eine aus durchsichtigem Kunststoff bestehende Trägerfolie (5) befestigt. Unter der Trägerfolie (5) befindet sich eine Reagenzzone (6) mit dem für die Bestimmung des Probeninhaltsstoffes notwendigen Reagenzien. Die Reagenzzone (6) ist so angeordnet, daß sie beim Niederdrücken der Trägerfolie (5) in Richtung Trägerfolie (4) mit der Transportschicht (3) in einen einen Flüssigkeitsübergang ermöglichenden Kontakt gebracht werden kann. Die Reagenzzone (6) besteht vorzugsweise aus einem quellfähigen oder/und saugfähigen Film, in den die für die Bestimmungsreaktion notwendigen Reagenzien eingearbeitet sind. Für den Creatin-Kinase-Nachweis können dies sein: Creatinphosphat, Adenosin-5'-diphosphat (ADP), ein zweiwertiges Metallkation, Glycerin, Glycerin-Kinase, Glycerinphosphat-Oxidase, Peroxidase, eine Verbindung der allgemeinen Formel I, gegebenenfalls ein Puffer sowie eine Substanz zur Inhibierung von Serummyokinase, wie beispielsweise P¹,P⁵-Di-(adenosin-5')-pentaphosphat. Als besonders vorteilhaft hat es sich speziell für die Bestimmung von Creatin-Kinase aus Vollblut erwiesen, wenn die Aktivierungsschicht (2) außer der Aktivatorensubstanz mit einer oder mehreren SH-Gruppen auch Creatinphosphat enthält und die Reagenzzone (6) die restlichen zur Bestimmung notwendigen Reagenzien.

Für die erfindungsgemäße Bestimmung von Creatin-Kinase aus Blut, Serum oder Plasma mittels Diarylimidazolen der allgemeinen Formel I hat es sich als ganz besonders vorteilhaft erwiesen, wenn das für den Enzymnachweis notwendige Reagenziensystem ein Detergens enthält. Vorzugsweise können nichtionische oder anionische Netzmittel eingesetzt werden. Besonders bevorzugt sind anionische Netzmittel, wie Dodecylsulfat, Tetradecylsulfat oder Hexadecylsulfat oder entsprechende Sulfonate, aber auch z. B. Diamylsulfosuccinat. Statt Netzmittel können aber auch andere Substanzen verwendet werden, die großvolumige Anionen, vorzugsweise starker Säuren, enthalten, wie beispielsweise Naphthalin-1,5-disulfonat, Tetrafluoroborat oder Perchlorat, wobei Naphthalin-1,5-disulfonat besonders bevorzugt ist.

Außerdem kann das für die Bestimmung von Creatin-Kinase notwendige Testsystem noch weitere Stoffe enthalten, wie z. B. einen Adenylat-Kinase-Inhibitor oder/und Komplexbildner, wie EDTA (Ethylendiamin-tetraacetat) oder EGTA (Ethylenglycol-bis(β-aminoethylether)tetraacetat).

Figur 2 unterscheidet sich von Figur 1 darin, daß die Aktivierungsschicht (2) in der Probenaufgabezone zwischen Schicht (1) zur Abtrennung korpuskulärer Bestandteile aus der Probenflüssigkeit und Transportschicht (3) angebracht ist.

Figur 3 zeigt einen Testträger, der wie die Vorrichtung aus Figur 2 aufgebaut ist, jedoch die für den Nachweis von Creatin-Kinase erforderlichen Reagenzien auf unterschiedlichen Trägern (8), (9) und (10) imprägniert enthält. Träger (10) entspricht der Reagenzzone (6) in Figuren 1 und 2. Die Träger (8) und (9) bestehen vorzugsweise aus einem dünnen saugfähigen Material, wie beispielsweise einem multifilen Gewebe oder Papier.

Zur Durchführung des erfindungsgemäßen Verfahrens mittels eines der in den Figuren dargestellten Testträgers, wird die zu untersuchende Probe als Flüssigkeit auf Schicht (1) aufgegeben. Dort werden korpuskuläre Bestandteile, wie beispielsweise Erythrozyten im Falle von Blut weitgehend zurückgehalten. Die Flüssigkeit gelangt im Falle eines Testträgers der Figuren 2 und 3 in die Aktivierungsschicht (2). Dort wird der zu bestimmende Probeninhaltsstoff, beispielsweise Creatin-Kinase durch die in der Aktivierungsschicht enthaltene SH-Gruppen tragende Verbindung reaktiviert. Die Flüssigkeit mit dem aktivierten Stoff, beispielsweise Creatin-Kinase füllt dann anschließend die Transportschicht (3). Im Falle eines Testträgers gemäß Figur 1 gelangt die Probenflüssigkeit durch Schicht (1) in die Transportschicht (3). Sie gelangt von dort durch Kapillarkräfte in die zwischen Transportschicht (3) und Trägerfolie (4) angeordnete Aktivierungsschicht (2), wo sie mit der Aktivatorsubstanz in Kontakt gelangt. Durch Druck der Trägerfolie (5) auf Schicht (3) kann dann die Bestimmungsreaktion gestartet werden. Die Flüssigkeit dringt durch den Druckkontakt in die Reagenzzone (6) (Figuren 1 und 2) bzw. in die Reagenzschichten (8), (9) und (10) (Figur 3). Die Reaktion wird anhand der Verfärbung in den Reagenzschichten durch die Trägerfolie (5) hindurch beobachtet.

Die vorgestellten Reagenzträger sehen eine Vorreaktion des Serums mit einer eine oder mehrere SH-Gruppen enthaltenden Verbindung zur Reaktivierung der Creatin-Kinase vor, wobei die Vorreaktion auch noch bis in die "lag phase" der Nachweiskaskade führen kann. Es ist auch möglich, daß diese Aktivierung und der Nachweis der Creatin-Kinase gleichzeitig erfolgt. Hierzu kann auf Schicht (2) verzichtet werden und die SH-Gruppen tragende Substanz in Reagenzzone (6) (Figuren 1 und 2) oder in einer der Reagenzschichten (8), (9) oder (10) (Figur 3) untergebracht werden.

Einige typische Konzentrationen der Reagenzien in den verschiedenen Schichten der Testträger nach Figuren 1, 2 und 3 sind:

| | |
|---|---|
| N-Acetylcystein | 0,01 - 0,8 µmol/cm², vorzugsweise 0,02 - 0,4 µmol/cm² |
| Creatinphosphat | 0,2 - 4 µmol/cm², vorzugsweise 0,4 - 2,4 µmol/cm² |
| Glycerin-Kinase | 0,1 - 15 U/cm², vorzugsweise 3 - 5 U/cm² |
| Glycerinphosphat-Oxidase | 0,1 - 10 U/cm², vorzugsweise 0,6 - 2 U/cm² |
| Meerrettich-Peroxidase | 0,1 - 20 U/cm², vorzugsweise 2 - 10 U/cm² |
| ADP | 0,002 - 0,1 µmol/cm², vorzugsweise 0,007 - 0,05 µmol/cm² |
| Glycerin erfindungsgemäßes | 0,01 - 0,5 µmol/cm², vorzugsweise 0,08 - 0,1 µmol/cm² |
| Diarylimidazol | 0,01 - 1 µmol/cm², vorzugsweise 0,04 - 0,2 µmol/cm² |
| Detergens | 0,01 - 8,0 µmol/cm², vorzugsweise 0,015 - 0,8 µmol/cm² |

Bei Durchführung des erfindungsgemäßen Verfahrens mittels Testträger gemäß Figuren 1 - 3 wird festgestellt, daß trotz hoher Konzentration an SH-Gruppen tragender Substanz kein Ausbleichen des oxidierten Diarylimidazols der Formel I erfolgt.

Insbesondere das testträgerbezogene Verfahren liefert zuverlässige und präzise Ergebnisse durch den Farbwechsel, welcher im sichtbaren Wellenlängenbereich zwischen etwa 560 und 680 nm registriert werden kann.

Weitere Vorteile des erfindungsgemäßen Verfahrens insbesondere bei Durchführung mittels eines Testträgers sind,
- daß keine Vorinkubation der Probe außerhalb des Testträgers erfolgen muß,
- daß vor allem bei der Untersuchung von Blut keine vorherige separate Abtrennung von Plasma oder Serum erforderlich ist,
- daß die Durchführung der Bestimmung innerhalb sehr kurzer Zeit, beispielsweise für Creatin-Kinase innerhalb von drei Minuten, möglich ist,
- daß auch sehr kleine Probenvolumina, etwa 30 µl untersucht werden können,
- daß bei Verwendung von Blut und Plasma oder Serum übereinstimmende Ergebnisse erhalten werden und
- daß auch in Gegenwart hoher Konzentrationen SH-Gruppen tragender Substanzen eine stabile Farbentwicklung eintritt.

Mit Ausnahme der Verbindung 2-(3′,5′-Di-tert.butyl-4′-hydroxyphenyl)-4(5)-(4˝-dimethylaminophenyl)-5(4)-methyl-(1H)-imidazolhydrochlorid sind Diarylimidazole der allgemeinen Formel I 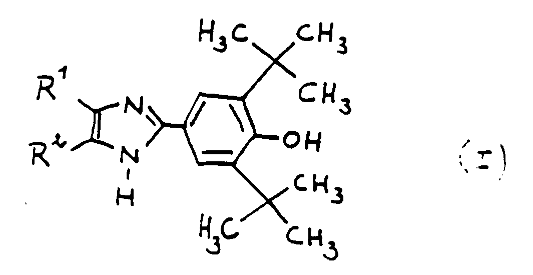
in der
R¹ einen Rest der allgemeinen Formel II 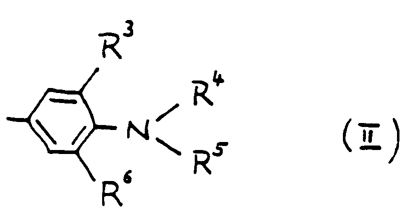
bedeutet, wobei
R³ und R⁶ Wasserstoff und
R⁴ und R⁵, die gleich oder verschieden sein können, Niederalkyl oder
R³/R⁴ oder/und R⁵/R⁶ jeweils zusammen einen Niederalkylenrest darstellen und
R² für einen Niederalkylrest steht
sowie deren Salze neu. Sie sind ebenfalls Gegenstand der vorliegenden Erfindung.

Die erfindungsgemäßen neuen Substanzen der Formel I werden in der Weise hergestellt, daß man ein α-Diketon der Formel III 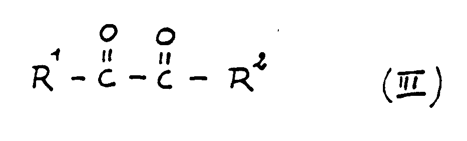
in der R¹ und R² die gleiche Bedeutung wie in Formel I besitzen mit einem Aldehyd der Formel IV 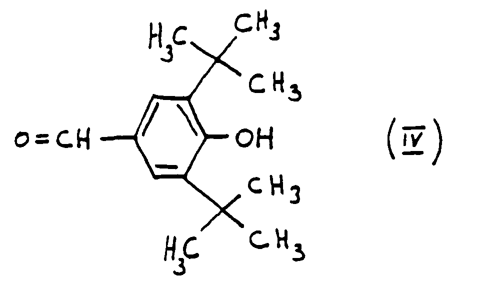
mit Ammoniak in saurer Lösung kondensiert und anschließend gegebenenfalls mit Säure nachbehandelt, wenn Salze der erfindungsgemäßen Verbindung erhalten werden sollen. Die Kondensationsreaktion wird vorzugsweise in essigsaurer Lösung durchgeführt, wobei bis zum Rückfluß erhitzt werden kann.

An den folgenden Beispielen soll die Erfindung näher erläutert werden.

### Beispiel 1

### 2-(3′,5′-Di-tert.-butyl-4′-hydroxyphenyl)-4-(5)-9˝-julolidyl-5-(4)-methyl-(1H)-imidazol

### 1.1 Julolidin

### (2,3,6,7-Tetrahydro-1H,5H-benzo[i,j]-chinolizin)

Die Herstellung der Verbindung erfolgte nach Organic Synthesis III, 504.

### 1.2 9-Julolidincarbaldehyd

### (1-(2,3,6,7-Tetrahydro-1H,5H-benzo-[ij]-chinolizin-9-yl) carbaldehyd)

Die Synthese des Produktes wird analog P.A.S. Smith und Tung Yin Yu, J. Org. Chem. 17, 1281 (1952) durchgeführt. Anstelle von N-Methylformanilid wird jedoch bei der Vilsmeier-Reaktion Dimethylformamid verwendet.

In einen 1 l Dreihalskolben mit Rührer, Rückflußkühler, Tropftrichter und Chlorkalziumrohr gibt man 220 g (1.32 mol) Julolidin, erhitzt im Wasserbad auf 60 °C bis zur Schmelze, löst diese durch Zugabe von 296 ml frisch destilliertem Dimethylformamid und tropft unter Rühren während 1.5 Stunden 120 ml, das sind ca. 226 g (1.31 mol), frisch destilliertes Phosphorylchlorid (Phosphoroxytrichlorid) unter Eiskühlung bei 20 - 30 °C zu. Nach 10 Minuten Nachrühren erhitzt man 3 Stunden auf 100 °C; das braune, ölige Reaktionsgemisch wird auf Raumtemperatur abgekühlt und unter Rühren in 1.36 kg Eis gegossen. Man rührt eine weitere Stunde nach und stellt den Ansatz über Nacht in den Kühlschrank. Die abgeschiedenen Kristalle werden abgesaugt und mit 100 ml Eiswasser gewaschen. Der gelblich-grüne Rückstand wird anschließend bei Raumtemperatur über Ätzkali im Vakuum bis zur Gewichtskonstanz getrocknet. Man erhält 255 g vom Schmelzpunkt 80 °C (Ausbeute 96 %).

DC: Kieselgel F 254 (E. Merck, Darmstadt, Bundesrepublik Deutschland), Laufmittel: Chloroform : Methanol 9:1
Detektion: Bromdämpfe
R_{f}-Wert: 0.89

Das Material wird ohne weitere Reinigung für die nächste Stufe verwendet. Durch Umkristallisieren aus wässrigem Ethanol erhält man ein Produkt vom Schmelzpunkt 83 °C.

### 1.3 1-(9-Julolidyl)propanol-(1)

### ([1-(2,3,6,7-Tetrahydro-1H,5H-benzo[ij]chinolizin-9-yl)-1-propanol]

In einen 4 l Dreihalskolben mit Rührer, Kühler und Chlorkalziumrohr gibt man 32.64 g (1.34 g Atom) Magnesiumspäne, überschichtet diese mit 231 ml absolutem Diethylether und tropft ohne Rührung 10 ml einer Lösung von 100.3 ml = 147 g (1.34 mol) Bromethan in 100 ml Diethylether zu; unter leichtem Erwärmen springt der Grignard an. Danach gibt man unter Rühren den Rest der Bromethan-Etherlösung so zu, daß der Ether am Sieden bleibt und erhitzt weitere 30 Minuten unter Rückfluß. Danach tropft man die Lösung von 135 g (0.67 mol) 9-Julolidincarbaldehyd in 1.25 l Tetrahydrofuran zu, wobei man die Tropfgeschwindigkeit so einstellt, daß der Ether am Sieden bleibt. Anschließend erhitzt man noch eine Stunde unter Rückfluß, kühlt auf Raumtemperatur ab, gibt unter Rühren portionsweise 191 g Eis zu, wobei Schäumen auftritt. Danach tropft man 382 ml 2 N Natronlauge zu, rührt 5 Minuten, läßt absitzen, dekantiert das Lösungsmittel ab und trocknet die Ether-Tetrahydrofuranphase über Natriumsulfat. Nach Zugabe von Aktivkohle saugt man ab und engt das Filtrat zur Trockne ein. Man verrührt den öligen Rückstand mit 1 l Ligroin und stellt zur Vervollständigung der eingeleiteten Kristallisation über Nacht in den Kühlschrank. Nach Absaugen und Waschen des Kristallisats mit 100 ml kaltem Ligroin erhält man nach mehrstündigem Trocknen im Vakuum über Molekularsieb 209 g beigefarbenes Carbinol, Schmelzpunkt 74 °C.

Durch Aufarbeiten der Mutterlauge können weitere 11.75 g des gleichen Produktes erhalten werden.

Gesamtausbeute: 71 % der Theorie
DC: Kieselgel F 254 (E. Merck, Darmstadt, Bundesrepublik Deutschland); Laufmittel: Toluol : Methanol 5 : 1
R_{f}-Wert = 0.3

### 1.4 1-(9-Julolidyl)propanon(1)

### [1-(2,3 6,7-Tetrahydro-1H,5H-benzo-[i,j]chinolizin-9-yl)-1-propanon]

Man löst 208.4 g (0.9 mol) 1-(9-Julolidyl)-propanol-1) im 6 l Dreihalskolben mit Rührer, Thermometer und Trockenrohr in 3.4 l Aceton, gibt 294.3 g (1.44 mol) Aluminiumtriisopropylat zu und kocht 4 Stunden unter kräftigem Rühren und Rückfluß, danach engt man am Rotationsverdampfer zur Trockne ein. Nach Zugabe von 2 l Essigester und 1 l 2 N Natronlauge rührt man 15 - 20 Minuten kräftig durch, läßt absitzen und dekantiert von der wässrigen schlammigen Aluminiumhydroxidfällung. Die Essigesterphase wird im Scheidetrichter abgetrennt und die wässrige Phase zweimal mit je 1 l Essigester 15 Minuten ausgerührt. Die Essigesterphasen werden vereinigt, über Natriumsulfat getrocknet und im Vakuum bei 40 °C eingeengt. Man erhält 305 g braunes Öl. Dieses wird in 500 ml Toluol-Essigester 1/1 gelöst, auf eine Kieselgelsäule (Merck 60), Höhe 120 cm; ⌀ 11 cm aufgetragen und mit Toluol/Essigester 15/1 fraktioniert. Man schneidet 35 Fraktionen à 500 ml und 4 Fraktionen à 1 l:
Fraktion 1 - 13 verworfen
14 - 33 eingeengt
34 - 39 verworfen
Nach Einengen der Fraktionen 14 - 33 im Vakuum bei 40 °C erhält man 101 g braunes Öl, das durch Anreiben mit 500 ml Ligroin zur Kristallisation gebracht wird. Nach Absaugen des Materials und Waschen des Rückstandes mit 100 ml kaltem Ligroin erhält man nach Trocknen über Diphosphorpentoxid-Molekularsieb 67 g (31.2 % der Theorie) schwach gelbe Kristalle, 1-(9-Julolidyl)-propanon(1), Schmelzpunkt 69 - 70 °C.

DC: Kieselgel F 254 (E. Merck, Darmstadt, Bundesrepublik Deutschland), Laufmittel: Toluol : Methanol 5:1
R_{f}-Wert 0.6
Die Mutterlauge (10 g) liefert nach säulenchromatographischer Trennung weitere 2.7 g = 1.26 % reines Keton, Schmelzpunkt 68 - 69 °C, Gesamtausbeute: 33.7 % der Theorie.

### 1.5 1-(9-Julolidyl)-2-brompropanon(1)

### [2-Brom-1-(2,3,6,7-tetrahydro-1H,5H-benzo[i,j]chinolizin-9-yl)-1-propanon] (MG 308.2)

67 g (0.29 mol) 1-(9-Julolidyl)propanon-(1) werden in 417 ml 47 %iger Bromwasserstoffsäure suspendiert auf 60 °C erwärmt, wobei teilweise Lösung eintritt und tropft innerhalb 20 Minuten eine Lösung von 15.8 ml Brom in 87 ml 47 %iger Bromwasserstoffsäure unter Rühren zu. Das Ausgangsmaterial geht dabei vollständig in Lösung. Man rührt noch eine Stunde bei 60 °C nach, kühlt auf 20 °C und läßt die Reaktionsmischung unter intensivem Rühren in eine Mischung von 900 ml Essigester und 1.9 l Wasser einlaufen. Nach 10 Minuten Rühren trennt man die Phasen im Scheidetrichter ab, die wässrige Phase wird noch zweimal mit je 700 ml Essigester durchgeschüttelt, die vereinigten Lösungsmittelextrakte über Natriumsulfat getrocknet, abgesaugt und am Rotationsverdampfer im Vakuum eingeengt. Das erhaltene Rohprodukt, 102 g braunes Öl, wird in 400 ml Isopropanol heiß gelöst und durch Abkühlen im Eisbad zur Kristallisation gebracht. Nach 14stündigem Einstellen in einem Kühlschrank saugt man die gebildeten Kristalle ab, trocknet diese 24 Stunden über Diphosphorpentoxid und erhält 83.5 g = 93.4 % der Theorie gelblich-grünes Bromketon vom Schmelzpunkt 83 - 84 °C.

DC: Kieselgel F 254 (E. Merck, Darmstadt, Bundesrepublik Deutschland), Laufmittel: Toluol : Essigester 5 : 1
R_{f}-Wert 0.31

### 1.6 1-(9-Julolidyl)-propandion-(1.2)

### [1-(2,3,6,7-Tetrahydro-1H-benzo[i,j]chinolizin-9-yl)-1.2-propandion]

In einen 1 l Dreihalskolben mit Thermometer und Rückflußkühler gibt man 112 ml (1.58 mol) Dimethylsulfoxid, 46.5 g (0.28 mol) Kaliumjodid und 29.7 g (0.28 mol) wasserfreies Natriumkarbonat, erhitzt unter Rühren auf 120 °C und gibt 86.3 g (0.28 mol) 1-(9-Julolidyl)-2-brompropanon(1) zu. Die Lösung wird eine Stunde bei 120 °C gerührt, wobei sich die gelbliche Lösung rotbraun färbt. Nach Abkühlen auf 20 °C gießt man die Reaktionslösung in 2.8 l gesättigte Kochsalzlösung. Die entstehende Suspension wird unter Eiskühlung 15 Minuten mit 2 l Ether angerührt. Die Etherphase wird abgetrennt und die wässrige Phase noch zweimal mit je 1 l Ether extrahiert. Die vereinigten Etherextrakte werden mit einmal 1 l gesättigter Kochsalzlösung, 1 l 5 %iger Natriumhydrogenkarbonatlösung und 1 l Wasser durchgeschüttelt, die Etherphase über Natriumsulfat getrocknet, mit Aktivkohle behandelt, abgesaugt und am Rotationsverdampfer im Vakuum zur Trockne gebracht. Das Rohprodukt, 64 g rötlich-braunes Öl wird in 300 ml Isopropanol heiß gelöst, abgekühlt und durch Animpfen und 48stündiges Einstellen in einem Kühlschrank zur Kristallisation gebracht. Man erhält 18.1 g Diketon, orangefarbene Kristalle. Die Mutterlauge wird eingeengt und das Rohprodukt 30 g, säulenchromatographisch gereinigt. An einer Kieselgelsäule (Merck 60), Höhe 11 cm, ⌀ 7.5 cm wird mit Toluol-Essigester eluiert. Es werden 70 Fraktionen à 80 ml geschnitten.

Die Fraktionen 1 - 43 und 57 - 70 werden verworfen. Die aufgearbeiteten Fraktionen 44 - 56 ergeben 6 g orangefarbenes Öl. Dieses wird in 40 ml Isopropanol heiß gelöst, die Lösung abgekühlt und nach Kühlen mit Eis abgesaugt. Man erhält weitere 5.2 g orangefarbenes Diketon. Die beiden Kristallisate werden vereinigt und nochmals auf 150 ml Isopropanol umkristallisiert. Nach Absaugen und Waschen des Filterkuchens mit wenig kaltem Isopropanol erhält man 22.7 g = 33.3 % 1-(9-Julolidyl)-propandion (1.2), orangefarbene Kristalle, Schmelzpunkt 111 - 113 °C.

DC: Kieselgel F 254 (E. Merck, Darmstadt, Bundesrepublik Deutschland), Laufmittel Toluol : Methanol 5 : 1
R_{f}-Wert = 0.32

### 1.7 2-(3′,5′-Di-tert.-butyl-4′-hydroxyphenyl)-4-(5)-(9˝-julolidyl)-5-(4)-methyl-(1H)-imidazol

### 2,6-Bis-(1,1-dimethylethyl)-4-[5-methyl-4-(2,3,6,7-tetrahydro-1H,5H-benzo[i,j]chinolizin-9-yl)imidazol-2-yl]-phenol

In einem 1 l Dreihalskolben mit Rührer, Kühler und Thermometer wird eine Mischung von 32.7 g (0.13 mol) 1-(9-Julolidyl)propandion-(1,2), 30.5 g(0.13 mol) 3,5-Di-tert.-butyl-4-hydroxybenzaldehyd und 100.2 g (1.3 mol) Ammoniumacetat in 300 ml p.a. Eisessig im Ölbad unter Argon oder Stickstoff als Schutzatmosphäre 3 Stunden unter Rückfluß erhitzt. Danach kühlt man auf 20 °C ab und tropft das Reaktionsgemisch unter kräftigem Rühren in 2 l Eiswasser ein. Nach Absaugen des ockerfarbenen Rückstandes wäscht man den Filterkuchen mit 1 l Wasser und trocknet das Produkt 24 Stunden über Diphosphorpentoxid und Kaliumhydroxid bei Raumtemperatur. (Sämtliche Arbeiten werden in abgedunkelten Räumen und aluminiumfoliebedeckten Apparaturen durchgeführt). Das Rohprodukt, 60 g ockerfarbener Rückstand wird mit 1.8 l Aceton unter Rühren am Rückfluß erhitzt, die beim Abkühlen erhaltenen Kristalle abgesaugt, mit 200 ml Aceton gewaschen und 16 Stunden im Vakuum über Molekularsieb und Diphosphorpentoxid getrocknet; danach rührt man zwecks Entfernung von Restaceton mit 500 ml Ligroin an, saugt ab und trocknet bei 40 °C an der laufenden Ölpumpe über Molekularsieb und Diphosphorpentoxid bis zur Gewichtskonstanz.

Man erhält 31 g (52 % der Theorie) hellbeigefarbene Kristalle von 2-(3′,5′-Di-tert.-butyl-4′-hydroxyphenyl)-4-(5)-(9˝-julolidyl)-5-(4)-methyl-(1H)imidazol. Schmelzpunkt > 260 °C (Zersetzung ab 300 °C).

DC: Fertigplatte Kieselgel F 254 (E. Merck, Darmstadt, Bundesrepublik Deutschland)
Laufmittel: Dioxan/Essigsäure/Toluol 20 : 80 : 72 R_{f}-Wert 0.35
Detektion: Chlor/Tetramethylbenzidin-Kaliumjodid

### Beispiel 2

### 2-(3′,5′-Di-tert.-butyl-4′-hydroxyphenyl)-4(5)-9˝-julolidyl-5 (4)-methyl-(1H) imidazol-hydrochlorid

### 2,6-Bis (1,1-dimethylethyl)-4-[5-methyl-4(2,3,6,7tetrahydro-1H,5H-benzo[i,j]chinolizin-9-yl)imidazol-2-yl]-phenol-hydrochlorid

Man suspendiert 20 g (0.437 mol) des in Beispiel 1 erhaltenen Imidazols in 50 ml Methanol und tropft unter Kühlung bei Raumtemperatur 30 ml gesättigter, etherischer Salzsäure zu. Dabei geht die Base in Lösung. Danach engt man bei Raumtemperatur im Vakuum ein und reibt den öligen Rückstand mit 200 ml Essigester an. Nach 15 Minuten Einstellen in ein Eisbad werden die gebildeten Kristalle scharf abgesaugt, mit wenig eiskaltem Essigester gewaschen und im Vakuum bei Raumtemperatur über Diphosphorpentoxid bis zur Gewichtskonstanz getrocknet. Man erhält 19.8 g (87.1 % der Theorie) rosafarbene Kristalle vom Schmelzpunkt 231 °C (Zers.).

R_{f}-Wert im Laufmittel Toluol : Dioxan : Eisessig 9 : 2.5 : 1 Kieselgel 60 (E. Merck, Darmstadt, Bundesrepublik Deutschland) = 0,38.

### Beispiel 3

### a) 2-(3′,5′-Di-tert.butyl-4′-hydroxyphenyl)-4(5)-(4˝-diethylaminophenyl-5(4)-methyl(1H)-imidazol-hydrochlorid

0,05 Mol 1-(4′-N-Diethylaminophenyl)-1,2-propandion (bzw. 1-[4-(Diethylamino)phenyl]-1,2-propandion) (erhalten analog 1-(9-Julolidyl)-1,2-propandion aus Beispiel 1, als Öl; DC: Kieselgel 60 (E. Merck, Darmstadt, Bundesrepublik Deutschland), Laufmittel: Toluol/Essigsäureethylester 4:1, R_{f}-Wert 0,8), werden mit 11,72 g (0,05 Mol) 3,5-Di-tert.butyl-4-hydroxybenzaldehyd und 38,5 g (0,5 Mol) Ammoniumacetat in 100 ml Eisessig 4 Stunden unter Argon als Schutzgas am Rückfluß erhitzt. Nach 12stündigem Stehen bei Raumtemperatur gießt man die dunkelgefärbte Reaktionsmischung auf 550 g Eis und 50 ml konzentrierte wässrige Ammoniaklösung, saugt den gebildeten Niederschlag scharf ab und nimmt das Rohprodukt in Essigsäureethylester auf. Nach Trocknen und Einengen führt man durch Lösen in Methanol und Zugabe von etherischer Chlorwasserstoffsäure in das Hydrochlorid über und erhält nach Umkristallisieren aus Isopropanol das stabile Endprodukt mit einer Ausbeute von 40 %.

Schmelzpunkt 220 °C (Zers.)
λₘₐₓ 672 nm (ε = 21 758)
DC: Kieselgel 60 (E. Merck, Darmstadt, Bundesrepublik
Deutschland), Laufmittel: Toluol/Methanol 5:1, R_{f} 0.33
Zur Herstellung des Diketons erforderliche Zwischenverbindungen:
1-[4-(Diethylamino)phenyl]-1-propanon (4′-N-Diethylamino)propiophenon), erhalten nach GB-A-911342 (Wellcome), analog A.W. Nineham, Soc. 635 (1952): Kp. 135-155°C, Schmelzpunkt: 44-45°C, Ausb. 55 % - DC: Kieselgel 60 (E. Merck, Darmstadt, BRD); Laufmittel: Toluol-Essigsäureethylester 4:1, R_{f} = 0,6
1-[4-(Diethylamino)phenyl]-2-brom-1-propanon: Ausb. 98 % d.Th., Schmelzpunkt 59-61°C; DC: Kieselgel 60 (E. Merck, Darmstadt, BRD), Laufmittel: Toluol-Essigsäureethylester 4:1, R_{f} = 0,7

### b) 2-(3′,5′-Di-tert.butyl-4′-hydroxyphenyl)-4(5)-(4˝-N-di-n-propyl-aminophenyl)-5(4)-methyl-(1H) imidazol-hydrochlorid

Diese Verbindung wird analog Beispiel 3 a) aus 1-(4′-Di-n-propyl-aminophenyl)-1,2-propandion (bzw. 1-(4-Di-n-propylaminophenyl)-1,2-propandion) (erhalten analog 1-(9′-Julolidyl)-1,2-propandion aus Beispiel 1, als Öl, DC: Kieselgel 60 (E. Merck, Darmstadt, Bundesrepublik Deutschland), Laufmittel: Methylenchlorid, R_{f} = 0,4) als Base mit einem Schmelzpunkt (Umkristallisation aus n-Heptan) von 33 - 34 °C; DC: Kieselgel 60 (E. Merck, Darmstadt, Bundesrepublik Deutschland), Laufmittel: Methylenchlorid, R_{f}-Wert: 0,2 hergestellt. Die Ausbeute beträgt 40 %. Analog Beispiel 3 a wird aus der Base das Hydrochlorid erhalten:
Schmelzpunkt: 246 - 248 °C
λₘₐₓ = 667 nm (ε = 20 700)
DC: Kieselgel 60 (E. Merck, Darmstadt, Bundesrepublik Deutschland), Laufmittel: Toluol/Methanol 5:1, R_{f}-Wert 0,38
Zur Herstellung des Diketons erforderliche Zwischenverbindungen:
4-Dipropylaminobenzaldehyd, hergestellt aus N,N-Dipropylanilin nach Duff. Furness, Soc. 1952, 1162: Kp (0,1 Torr) 145-148°C, DC Kieselgel 60 (E. Merck, Darmstadt, BRD), Laufmittel: n-Heptan-Methylethylketon 2:1, R_{f} = 0,57
1-[4-(Di-n-propylamino)phenyl]-1-propanol: Ausbeute 90 % d.Th., Öl. DC Kieselgel 60 (E. Merck, Darmstadt, BRD) Laufmittel: Toluol/Essigsäureethylester 4:1, R_{f} = 0,46.
1-[4-(Di-n-propylamino)phenyl]-1-propanon: Ausbeute 48 % d.Th., Schmelzpunkt 41-43°C, DC Kieselgel 60 (E. Merck, Darmstadt, BRD) Laufmittel: Toluol/Essigsäureethylester 4:1, R_{f} = 0,7.
1-[4-(Di-n-propylamino)phenyl]-2-brom-1-propanol: Ausbeute 87 % d.Th., gelbes Öl. DC Kieselgel 60 (E. Merck, Darmstadt, BRD) Laufmittel: Toluol/Essigsäureethylester 4:1, R_{f} = 0,8.

c) Anstelle der direkten Überführung der 1-[4-(Dialkylamino)phenyl]-2-brom-1-propanone in die betreffenden 1-[4-(Dialkylamino)phenyl]-1,2-propandione kann auch der Weg über die entsprechende 2-Acetoxy- bzw. 2-Hydroxyverbindung mit nachfolgender Oxidation durch Kupfersalze, vorzugsweise Kupferacetat, gewählt werden. Die Methode soll im nachfolgenden an einem allgemeinen Beispiel erläutert werden:

### Überführung von 1-[4-(Dialkylamino)phenyl]-2-brom-1-propanonen in 1- [4-(Dialkylamino)phenyl]-1,2-propandione

0,1 mol 1-[4-(Dialkylamino)phenyl]-2-brom-1-propanon werden in 170 ml Eisessig mit 0,8 mol wasserfreiem Natriumacetat 3 Stunden bei 130°C (Badtemperatur) gerührt. Danach gießt man auf 900 ml Wasser und trennt das gebildete Öl durch Ausschütteln mit 3 x 200 ml Methylenchlorid ab. Nach Waschen der organischen Phase mit Wasser und Einengen im Vakuum erhält man die betreffende Acetoxyverbindung in nahezu quantitativer Ausbeute.

Dieses Rohprodukt wird mit 88 ml 2 n Salzsäure 2 Stunden bei 80°C gerührt. Nach Abkühlen wird durch vorsichtige Zugabe von 30 g Natriumhydrogencarbonat neutralisiert und das gebildete 1-[4-(Dialkylamino)phenyl]-2-hydroxy-1-propanon mit 3 x 100 ml Methylenchlorid ausgeschüttelt. Die vereinigten organischen Phasen werden mit 200 ml Wasser geschüttelt, getrocknet und eingeengt. Ausbeute quantitativ. Das erhaltene Produkt wird mit 51 g (0,25 mol) Kupfer(II)acetat . H₂O, 200 ml Ethanol und 100 ml Wasser 6,5 Stunden bei 90°C (Badtemperatur) gerührt. Die gebildete Fällung wird abgesaugt und der Rückstand mit etwas Ethanol/Wasser = 2:1 gewaschen. Das Filtrat wird mit 500 ml Wasser verdünnt und das ausgeschiedene Öl mit 3 x 200 ml Methylenchlorid ausgeschüttelt. Nach Waschen der vereinigten organischen Phasen mit Wasser, Trocknen und Einengen wird der Eindampfrückstand über 900 g Kieselgel 60 (Merck, Darmstadt, BRD) an einer Säule, Durchmesser 7,5 cm, Schichthöhe 45 cm mit Methylenchlorid chromatographiert.

Die jeweiligen Zwischenverbindungen sind hierbei:
aa) 1-[4-(Diethylamino)phenyl]-2-acetoxy-1-propanon: Ausbeute 90 % d.Th., Schmelzpunkt 75-77°C, DC: Kieselgel 60 (E. Merck, Darmstadt, BRD), Laufmittel: Toluol/Essigsäureethylester 4:1, R_{f} = 0,5.
   1-[4-(Diethylamino)phenyl]-2-hydroxy-1-propanon: Ausbeute 79.5 % d.Th., amorph, DC: Kieselgel 60 (E. Merck, Darmstadt, BRD). Laufmittel: Toluol/Essigsäureethylester 4:1, R_{f} = 0,4.
bb) 1-[4-(Di-n-propylamino)phenyl]-2-acetoxy-1-propanon: Ausbeute 92,2 % d.Th., braunes Öl. DC Kieselgel 60 (E. Merck, Darmstadt, BRD) Laufmittel: Toluol/Essigsäureethylester 4:1, R_{f} = 0,5.
   1-[4-(Di-n-propylamino)phenyl]-2-hydroxy-1-propanon: Ausbeute 98,9 % d.Th., Öl. DC Kieselgel 60 (E. Merck, Darmstadt, BRD) Laufmittel: Toluol/Essigsäureethylester 4:1, Rf = 0,42.

### Beispiel 4

### Testträger zur Bestimmung von Creatin-Kinase

Es wird ein Testträger gemäß Figur 1 hergestellt. Schicht (1) ist ein 0,5 mm dickes Glasfaservlies aus Glas der Qualität 753 Typ 108A (Johns Mansville, USA) mit einem Flächengewicht von 50 - 60 g/m² und der Größe 6 mm x 6 mm.

Transportschicht 83) ist ein 0,15 mm dickes Glasfaservlies aus Glas der Qualität 753 Typ 106 (Johns Mansville, USA), verstärkt mit 10% Polyesterfaser, einem Flächengewicht von 20-25 g/m² und der Größe 16 mm x 6mm.

Aktivierungsschicht (12) besteht aus Polyestergewebe 14/100 der Firma SST/Schweiz mit einer Saugkapazität von ca. 55 ml/m² und einer Luftdurchlässigkeit von ca. 5000 l/m² sec. Dieses Material wird getränkt mit einer auf pH 8,0 eingestellten wässrigen Lösung enthaltend 200 mmol/l Creatin-Phosphat, 30 mmol/l N-Acetylcystein und 0,05 Gew.-% Triton® x-100. Das Gewebe hat die Ausmaße 12 mm x 6 mm.

Trägerfolie (5) ist transparent und besteht aus Polycarbonat. Sie ist 15 mm lang und 6 mm breit.

Reagenzzone (6) wird hergestellt, indem Propiofan® (BASF Ludwigshafen, Bundesrepublik Deutschland), Kieselgur, Korngrößenverteilung 100% <48 µm, 95% <32 µm, 70 % <16µm, 25-45 % <8µm (Eagle Picker, USA), Titandioxid Typ p-25 hochdispers (Degussa, Bundesrepublik Deutschland) und Xanthan (Kelco Oil International, Hamburg, Bundesrepublik Deutschland) im Gewichtsverhältnis 1:2:0,8:0,04 gemischt und zu einer viskosen Menge verarbeitet werden. In der auf Kieselgur bezogenen doppelten Menge Phosphatpuffer pH 6,3 werden gelöst:

| | |
|---|---|
| Ascarbat-Oxidase | 200 U/g |
| Glycerin-Kinase | 300 U/g |
| α-Glycerinphosphat-Oxidase | 50 U/g |
| Meerrettich-Peroxidase | 60 U/g |
| Glycerin | 8 µmol/g |
| Magnesiumaspartat | 10 µmol/g |
| Adenosindiphosphat | 30 µmol/g |
| P¹,P⁵-Di(adenosin-5')-pentaphospat | 2,7 nmol/g |
| Natriumdodecylsulfat | 8 µmol/g |
| Triton® X-100 | 2 Gew.-% |
| 2-(3',5'-Di-tert.-butyl-4'-hydroxyphenyl)-4(5)-(9''-julolidyl)-5(4)-methyl-(1H)-imidazolhydrochlorid | 40 µmol/g |

Die viskose Masse und die Phosphatpufferlösung werden zu einer fließfähigen Masse verarbeitet und im Rakelbeschichtungsverfahren in einer Schichtdicke von 200 µ auf die Polycarbonatfolie aufgetragen.

Die einzelnen Schichten werden mittels Schmelzkleber auf der Trägerfolie (4) wie in Figur 1 gezeigt, befestigt.

Zur Bestimmung von Creatin-Kinase in Blut werden 30 µl Blut, Plasma oder Serum auf die Abtrennschicht (1) gegeben. Nach 60 Sekunden wird die Trägerfolie (5) mit der Reagenzzone (6) auf die Transportschicht (3) gedrückt und die Farbänderung in der Reagenzzone (6) in den nächsten zwei Minuten reflexionsphotometrisch gemessen. Mittels der gemessenen Reflexionswerte kann aus einer Eichkurve die zu bestimmende Creatin-Kinase-Aktivität abgelesen werden.

Wie aus Tabelle 1 ersichtlich, werden für Blutproben unterschiedlichen Hämatokrits aber gleichen Creatin-Kinase-Gehalts gut übereinstimmende Resultate für die Creatin-Kinase-Aktivität erhalten.

**Tabelle 1**

| Hämatokrit in % | Creatin-Kinase-Aktivität in U/l |
|---|---|
| 20 | 71 |
| 30 | 74,5 |
| 40 | 73,5 |
| 44 | 73,5 |
| 50 | 71 |

Aus Tabelle 2 ist ersichtlich, daß bei Proben des gleichen Spenders in den Grenzen der Meßfehlergenauigkeit auch übereinstimmende Resultate für Plasma und Vollblut erhalten werden. Der Hämatokrit betrug jeweils 44 %.

**Tabelle 2**

| Spender | Creatin-Kinase-Aktivität in U/l | |
|---|---|---|
| | Blut | Plasma |
| 1 | 73,5 | 72 |
| 2 | 745 | 740 |
| 3 | 1610 | 1625 |

Es ist zu beachten, daß bei Verwendung der gleichen Volumina Plasma und Blut aufgrund des Hämatokrits mit der Blutprobe auf dem Testträger eine bis zu 4fach höhere N-Acetylcystein-Konzentration eingestellt wird als mit Plasma. Die Ergebnisse der Tabelle 2 belegen also, daß auch bei erhöhter Konzentration an SH-Gruppenhaltiger Substanz in der Probenflüssigkeit zuverlässige Resultate erhalten werden. Das gleiche Fazit ist aus den Ergebnissen der Tabelle 3 zu ziehen, in der unterschiedliche Probenvolumina (Serum) auf den Testträger aufgebracht wurden und die Creatin-Kinase-Aktivität gemessen wurde.

**Tabelle 3**

| Probenvolumen in µl | Creatin-Kinase-Aktivität in U/l bei Serum | |
|---|---|---|
| | Spender 1 | Spender 2 |
| 28 | 92 | 2127 |
| 30 | 92 | 2122 |
| 32 | 96 | 2216 |
| 35 | 94 | 2248 |

Die Ergebnisse zeigen, daß der erfindungsgemäße Testträger zur Bestimmung von Creatin-Kinase in einem weiten Bereich volumenunabhängig ist und sowohl bei niedrigen als auch bei hohen N-Acetylcystein-Konzentrationen übereinstimmende Resultate liefert.

### Beispiel 5

### Vergleich von Diarylimidazolen in Testträgern gemäß Figur 1

A) Es werden Testträger wie in Beispiel 4 beschrieben, hergestellt. Sie unterscheiden sich jeweils nur durch das eingesetzte Diarylimidazol.
Testträger 1 enthält 2-(3′,5′-Dimethoxy-4′-hydroxyphenyl)-4(5)-(4˝-dimethylaminophenyl)-5(4)-methyl-(1H)-imidazol-hydrochlorid.
Testträger 2 enthält 2-(3′,5′-Di-tert.-butyl-4′-hydroxyphenyl)-4(5)-(4˝-dimethylaminophenyl)-5(4)-methyl-(1H)-imidazol-hydrochlorid.
Testträger 3 enthält 2-(3′,5′-Di-tert.-butyl-4′-hydroxyphenyl)-4(5)-(4˝-diethylaminophenyl)-5(4)-methyl-(1H)-imidazolhydrochlorid.
Testträger 4 enthält 2-(3′,5′-Di-tert.butyl-4′-hydroxyphenyl)-4(5)-4˝-di-n-propylaminophenyl)-5-(4)-methyl-(1H)-imidazolhydrochlorid.
Testträger 5 enthält 2-(3′,5′-Di-tert.butyl-4′-hydroxyphenyl)-4(5)-(4˝-aminophenyl)-5(4)-methyl-(1H)-imidazol-hydrochlorid.
Testträger 6 ist identisch mit dem in Beispiel 4 hergestellten erfindungsgemäßen Testträger.
Bei Verwendung von jeweils 30 µl Serum erhält man die in Tabelle 4 wiedergegebenen Ergebnisse.

**Tabelle 4**

| Testträger Nr. | Creatin-Kinase-Aktivität in U/l |
|---|---|
| 1 | weniger als 0,1 |
| 2 | 2061 |
| 3 | 2043 |
| 4 | 2087 |
| 5 | 430 |
| 6 | 2100 |

Aus den vorstehenden Resultaten wird deutlich, daß nur die erfindungsgemäßen Diarylimidazole in den Testträgern 2, 3, 4 und 6 verwendet werden können, ohne daß das gebildete Oxidationsprodukt des Indikators von N-Acetylcystein ausgebleicht wird (wie bei den Testträgern 1 und 5)
B) Es werden Testträger wie unter A) beschrieben hergestellt, die aber alle kein N-Acetylcystein enthalten. Bei Verwendung von jeweils 30 µl Serum werden die in Tabelle 5 wiedergegebenen Ergebnisse erhalten.

**Tabelle 5**

| Testträger Nr. | Creatin-Kinase-Aktivität in U/l |
|---|---|
| 1 | 1071 |
| 2 | 1100 |
| 3 | 980 |
| 4 | 1020 |
| 5 | 957 |
| 6 | 1032 |

Ohne N-Acetylcystein findet keine Reaktivierung oxidierter Creatin-Kinase statt. Testträger 1 bis 6 liefern vergleichbare Ergebnisse, die aber in ihrem Wert alle niedriger sind als die in Tabelle 4 wiedergegebenen.

Bei Vergleich mit den Ergebnissen der Tabelle 4 ist festzustellen, daß die Diarylimidazole der Testträger 1 und 5 offensichtlich durch N-Acetylcystein gestört werden. Die erfindungsgemäßen Diarylimidazole in den Testträgern 2, 3, 4 und 6 eignen sich dagegen sehr gut für Messungen in Anwesenheit von N-Acetylcystein.

## Patentansprüche

1. Verwendung eines Diarylimidazols der allgemeinen Formel I 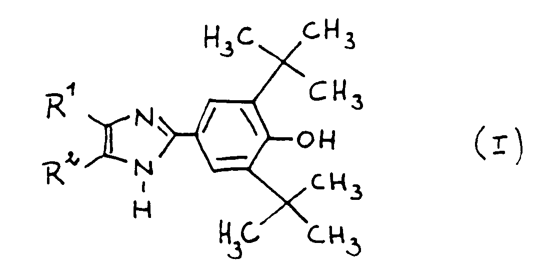 in der R¹ einen Rest der allgemeinen Formel II 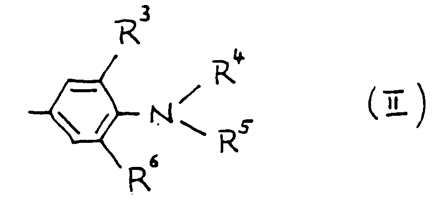 bedeutet, wobei
R³ und R⁶ Wasserstoff und
R⁴ und R⁵, die gleich oder verschieden sein können,
C₁ - C₆ Alkyl oder
R³/R⁴ oder/und R⁵/R⁶ jeweils zusammen einen C₃- oder C₄-Alkylenrest darstellen und
R² für einen C₁ - C₆-Alkylrest steht
oder dessen Salz einer starken Säure
als Redoxindikator.

2. Verwendung eines Diarylimidazols gemäß Anspruch 1 in Gegenwart von einer eine oder mehrere SH-Gruppen enthaltenden Substanz als Redoxindikator.

3. Verwendung eines Diarylimidazols gemäß Anspruch 1 zur Bestimmung von Wasserstoffperoxid.

4. Verfahren zur Bestimmung eines Probeninhaltsstoffes, wobei eine Probe mit einer eine oder mehrere SH-Gruppen enthaltenden Substanz versetzt und mit einem Testsystem kontaktiert wird, das mit dem in der Probe zu bestimmenden Inhaltsstoff Wasserstoffperoxid bildet, welches kolorimetrisch bestimmt wird, dadurch gekennzeichnet, daß das gebildete Wasserstoffperoxid in Gegenwart von Peroxidase und der anfänglich zugesetzten eine oder mehrere SH-Gruppen enthaltende Substanz mittels eines Diarylimidazols der allgemeinen Formel I 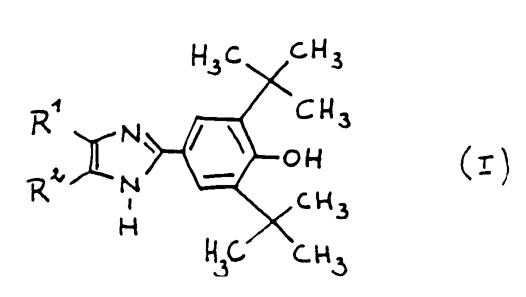 in der R¹ einen Rest der allgemeinen Formel II 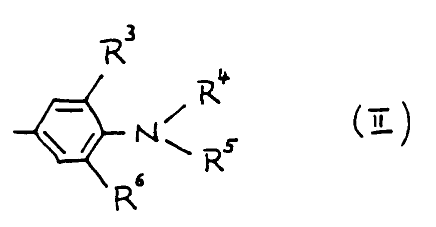 bedeutet, wobei
R³ und R⁶ Wasserstoff und
R⁴ und R⁵, die gleich oder verschieden sein können,
C₁-C₆-Alkyl oder
R³/R⁴ oder/und R⁵/R⁶ jeweils zusammen einen C₃- oder C₄-Alkylenrest darstellen und
R² für einen C₁ -C₆-Alkylrest steht
oder dessen Salz einer starken Säure
bestimmt wird.

5. Verfahren gemäß Anspruch 4 zur Bestimmung von Creatin-Kinase.

6. Verfahren gemäß einem der Ansprüche 4 oder 5, dadurch gekennzeichnet, daß als SH-Gruppen enthaltende Substanz N-Acetylcystein und als Diarylimidazol eine Verbindung gemäß Anspruch 1 eingesetzt wird, wobei
R¹ einen p-Dimethylamino-phenylrest,
einen p-Diethylamino-phenylrest,
einen p-Di-n-propylamino-phenylrest oder
einen Julolidinrest und
R² eine Methylgruppe darstellen.

7. Verfahren gemäß einem der Ansprüche 4 - 6, dadurch gekennzeichnet, daß die Bestimmung des Probeninhaltsstoffes in Gegenwart von 2 - 20 mmol/l einer eine oder mehrere SH-Gruppen enthaltenden Substanz erfolgt.

8. Mittel zur Bestimmung eines Probeninhaltsstoffes enthaltend eine eine oder mehrere SH-Gruppen tragende Substanz, ein Testsystem zum Nachweis des Probeninhaltsstoffes durch Bildung von Wasserstoffperoxid und einen Redoxindikator zur Bestimmung von Wasserstoffperoxid in Gegenwart von Peroxidase, dadurch gekennzeichnet, daß der Redoxindikator ein Diarylimidazol der allgemeinen Formel I 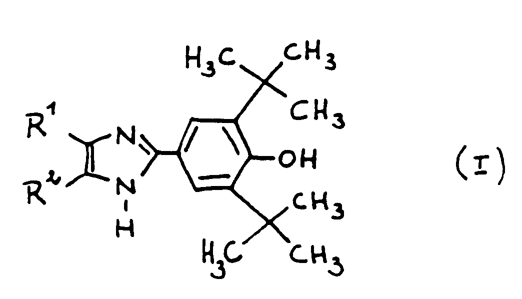 in der R¹ einen Rest der allgemeinen Formel II 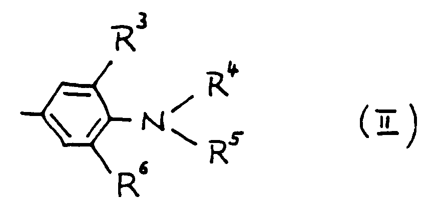 bedeutet, wobei
R³ und R⁶ Wasserstoff und
R⁴ und R⁵, die gleich oder verschieden sein können, C₁-C₆-Alkyl oder
R³/R⁴ oder/und R⁵/R⁶ jeweils zusammen einen C₃- oder C₄-Alkylenrest darstellen und
R² für einen C₁-C₆ -Alkylrest steht
oder dessen Salz einer starken Säure ist.

9. Mittel gemäß Anspruch 8 zur Bestimmung von Creatin-Kinase.

10. Mittel gemäß einem der Ansprüche 8 oder 9, dadurch gekennzeichnet, daß die zur Bestimmung des Probeninhaltsstoffes erforderlichen Substanzen auf einem festen Reagenzträger vorliegen.

11. Diarylimidazol der allgemeinen Formel I 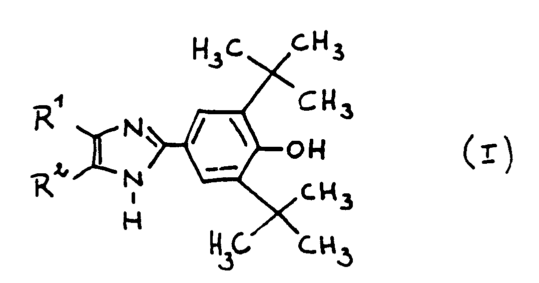 in der R¹ einen Rest der allgemeinen Formel II 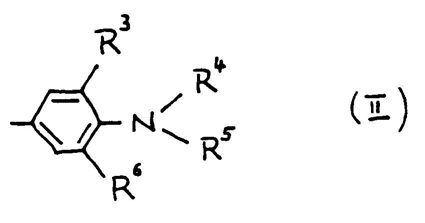 bedeutet, wobei
R³ und R⁶ Wasserstoff und
R⁴ und R⁵, die gleich oder verschieden sein können, C₁-C₆-Alkyl oder
R³/R⁴ oder/und R⁵/R⁶ jeweils zusammen einen C₃- oder C₄-Alkylenrest
darstellen und
R² für einen C₁ - C₆-Alkylrest steht
sowie dessen Salz einer starken Säure
mit Ausnahme der Verbindung 2-(3',5'-Di-tert.butyl-4'-hydroxyphenyl)-4(5)-4''-dimethylaminophenyl)-5(4)-methyl-(1H)-imidazol-hydrochlorid

12. Diarylimidazol gemäß Anspruch 1, dadurch gekennzeichnet, daß R¹ einen p-Diethylamino-phenylrest, einen p-Di-n-propylaminophenylrest oder einen Julolidinrest und R² eine Methylgruppe darstellen.

13. 2-(3',5'-Di-tert.-butyl-4'-hydroxyphenyl)-4(5)-(9''-julolidyl)-5(4)-methyl-(1H)-imidazol oder dessen Salz gemäß Anspruch 1.

14. Verfahren zur Herstellung einer Verbindung gemäß Anspruch 1, dadurch gekennzeichnet, daß man
ein α-Diketon der Formel III 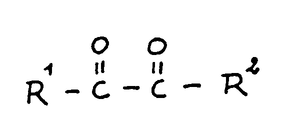 in der R¹ und R² die gleiche Bedeutung wie in Anspruch 1 besitzen mit einem Aldehyd der Formel IV 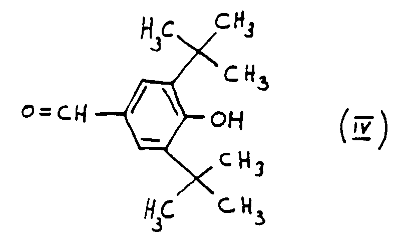 mit Ammoniak in saurer Lösung kondensiert und anschließend gegebenenfalls mit Säure nachbehandelt.

## Claims

1. Use of a diarylimidazole of the general formula I 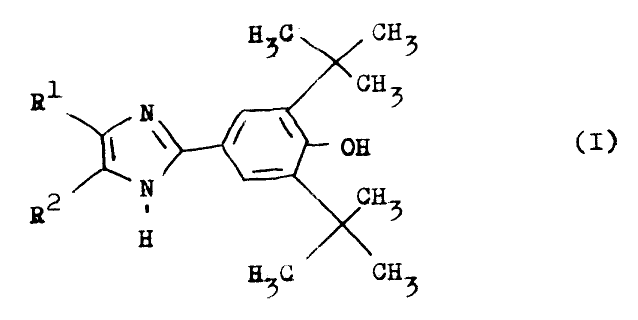 in which R¹ signifies a radical of the general formula II 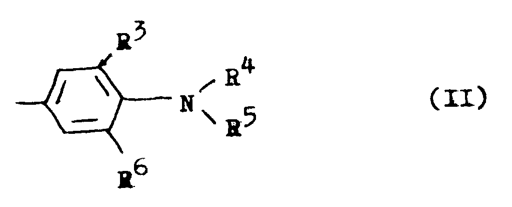 whereby R³ and R⁶ represent hydrogen, R⁴ and R⁵, which can be the same or different, represent C₁-C₆-alkyl or R³/R⁴ and/or R⁵/R⁶ in each case together represent C₃- or C₄-alkylene radicals and R² stands for a C₁-C₆-alkyl radical, or its salt of a strong acid as redox indicator.

2. Use of a diarylimidazole according to claim 1 in the presence of one or more SH group-containing substances as redox indicator.

3. Use of a diarylimidazole according to claim 1 for the determination of hydrogen peroxide.

4. Process for the determination of a simple component material, whereby a sample is mixed with a substance containing one or more SH groups and is contacted with a test system which, with the component material to be determined in the sample, forms hydrogen peroxide which is determined colorimetrically, characterized in that the hydrogen peroxide formed is determined in the presence of peroxidase and of the initially added substance containing one or more SH groups by means of a diarylimidazole of the general formula I 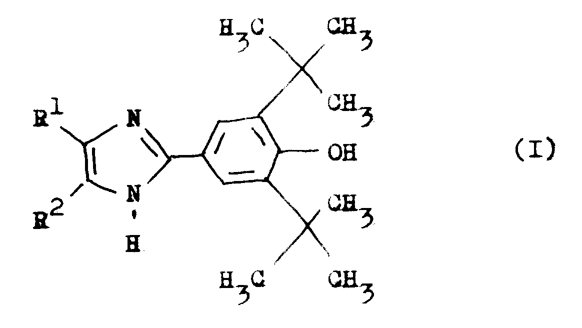 in which R¹ signifies a radical of the general formula II 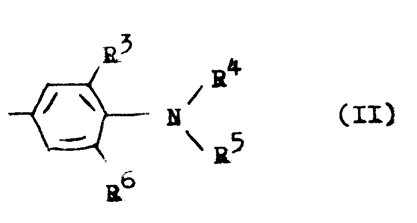 whereby R³ and R⁶ represent hydrogen and R⁴ and R⁵, which can be the same or different represent C₁-C₆-alkyl or R³/R⁴ and/or R⁵/R⁶ in each case together represent a C₃- or C₄-alkylene radical and R² stands for a C₁-C₆-alkyl radical or its salt of a strong acid.

5. Process according to claim 4 for the determination of creatine kinase.

6. Process according to one of claims 4 or 5, characterised in that N-acetylcysteine is used as SH group-containing substance and a compound according to claim 1 as diarylimidazole, whereby R¹ represents a p-dimethylaminophenyl radical, a p-diethylaminophenyl radical, a p-di-n-propylaminophenyl radical or a julolidine radical and R² a methyl group.

7. Process according to one of claims 4 - 6, characterised in that the determination of the sample component material takes place in the presence of 2 - 20 mmol/l of a substance containing one or more SH groups.

8. Agent for the determination of a sample component material containing a substance carrying one or more SH groups, a test system for the detection of the sample component material by formation of hydrogen peroxide and a redox indicator for the determination of hydrogen peroxide in the presence of peroxidase, characterised in that the redox indicator is a diarylimidazole of the general formula I 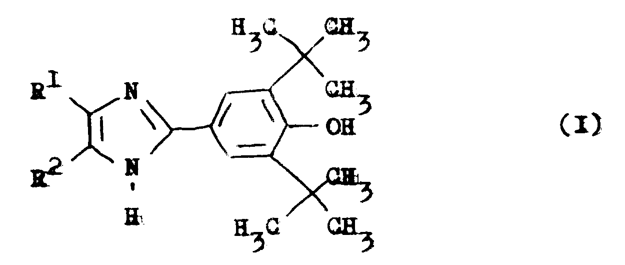 in which R¹ signifies a radical of the general formula II 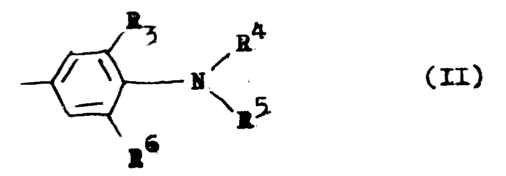 whereby R³ and R⁶ represent hydrogen and R⁴ and R⁵, which can be the same or different, represent C₁-C₆-alkyl or R³/R⁴ and/or R⁵/R⁶ in each case together represent a C₃- C₄-alkylene radical and R² stands for a C₁-C₆-alkyl radical or its salt of a strong acid.

9. Agent according to claim 8 for the determination of creatine kinase.

10. Agent according to one of claims 8 or 9, characterised in that the substances necessary for the determination of the sample component material are present on a solid reagent carrier.

11. Diarylimidazoles of the general formula I 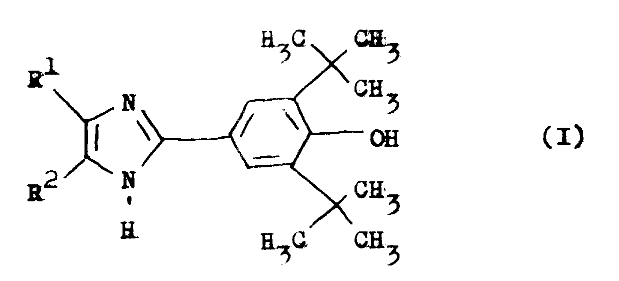 in which R¹ is a radical of the general formula II 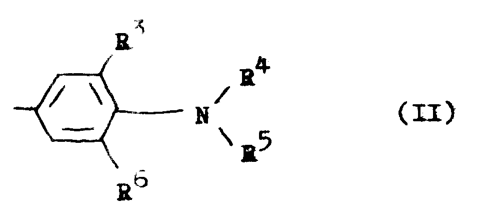 whereby R³ and R⁶ represent hydrogen and R⁴ and R⁵, which can be the same or different represent C₁-C₆-alkyl or R³/R⁴ and/or R⁵/R⁶ in each case together represent a C₃- or C₄-alkylene radical and R² stands for a C₁-C₆-alkyl radical, as well as its salts of a strong acid, with the exception of the compound 2-(3',5'-di-tert.-butyl-4'-hydroxyphenyl)-4(5)-4''-dimethylaminophenyl)-5(4)-methyl-(1H)-imidazole hydrochloride.

12. Diarylimidazoles according to claim 1, characterised in that R¹ represents a p-diethylaminophenyl radical, a p-di-n-propylaminophenyl radical or a julolidine radical and R² a methyl group.

13. 2-(3',5'-di-tert.-butyl-4'-hydroxyphenyl)-4(5)-(9''-julolidyl)-5(4)-methyl-(1H)-imidazole and its salt according to claim 1.

14. Process for the preparation of a compound according to claim 1, characterised in that one condenses an α-diketone of the formula III 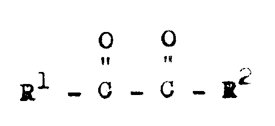 in which R¹ and R² possess the same meaning as in claim 1, with an aldehyde of the formula IV 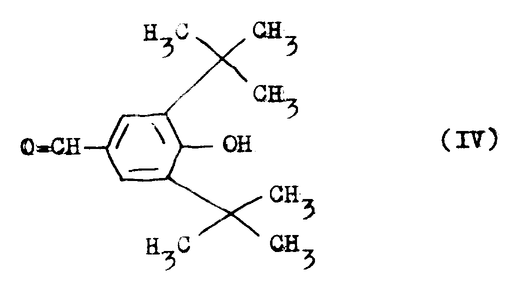 with ammonia in acid solution and subsequently possibly after-treats with acid.

## Revendications

1. Utilisation d'un diarylimidazole de formule générale I 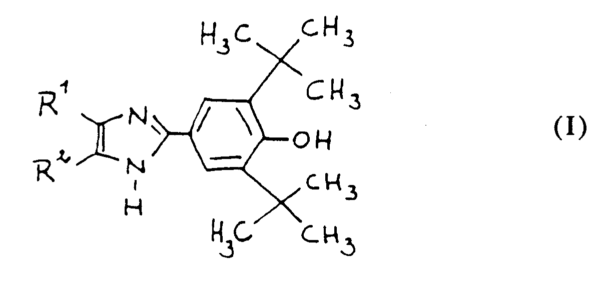 dans laquelle R¹ représente un groupe de formule générale II 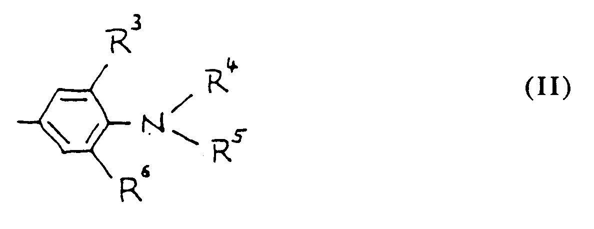 dans laquelle
R³ et R⁶ représentent un atome d'hydrogène et
R⁴ et R⁵, qui peuvent être identiques ou différents, représentent un groupe alkyle en C₁-C₆, ou
R³/R⁴ et/ou R⁵/R⁶ représentent chaque fois ensemble un groupe alkylène en C₃ ou en C₄, et
R² représente un groupe alkyle en C₁-C₆,
ou d'un de ses sels formés avec un acide fort, comme indicateur redox.

2. Utilisation d'un diarylimidazole selon la revendication 1, en présence d'une substance contenant un ou plusieurs groupes SH, comme indicateur redox.

3. Utilisation d'un diarylimidazole selon la revendication 1, pour la détermination de peroxyde d'hydrogène.

4. Procédé de détermination d'une substance contenue dans un échantillon, dans lequel on ajoute à un échantillon une substance contenant un ou plusieurs groupes SH et on met l'échantillon en contact avec un système de test qui forme, avec la substance à déterminer contenue dans l'échantillon, du peroxyde d'hydrogène, qui est déterminé par dosage colorimétrique, caractérisé en ce que le peroxyde d'hydrogène formé est déterminé en présence de peroxydase et de la substance ajoutée initialement, contenant un ou plusieurs groupes SH, à l'aide d'un diarylimidazole de formule générale I 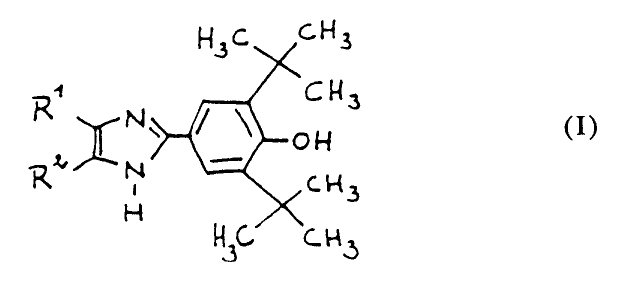 dans laquelle R¹ représente un groupe de formule II 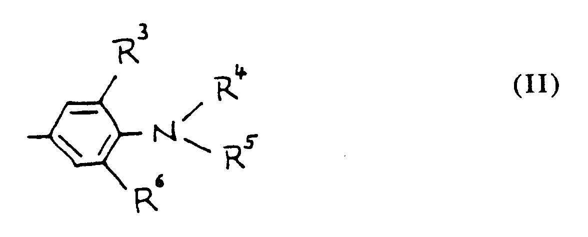 dans laquelle
R³ et R⁶ représentent un atome d'hydrogène et
R⁴ et R⁵, qui peuvent être identiques ou différents, représentent un groupe alkyle en C₁-C₆, ou
R³/R⁴ et/ou R⁵/R⁶ représentent chaque fois ensemble un groupe alkylène en C₃ ou C₄, et
R² représente un groupe alkyle en C₁-C₆,
ou d'un de ses sels formés avec un acide fort.

5. Procédé selon la revendication 4, pour la détermination de créatine-kinase.

6. Procédé selon l'une quelconque des revendications 4 ou 5, caractérisé en ce que, comme substance contenant des groupes SH, on utilise la N-acétyl-cystéine et comme diarylimidazole, on utilise un composé selon la revendication 1, dans lequel
R¹ représente un groupe p-diméthylamino-phényle,
un groupe p-diéthylamino-phényle,
un groupe p-di-n-propylamino-phényle ou
un groupe julolidine et
R² représente un groupe méthyle.

7. Procédé selon l'une quelconque des revendications 4 à 6, caractérisé en ce que la détermination de la substance contenue dans l'échantillon est réalisée en présence de 2 - 20 mmoles/l d'une substance contenant un ou plusieurs groupes SH.

8. Agent pour la détermination d'une substance contenue dans un échantillon, contenant une substance portant un ou plusieurs groupes SH, un système de test pour la détection, par formation de peroxyde d'hydrogène, de la substance contenue dans l'échantillon, et un indicateur redox pour la détermination du peroxyde d'hydrogène en présence de peroxydase, caractérisé en ce que l'indicateur redox est un diarylimidazole de formule générale I 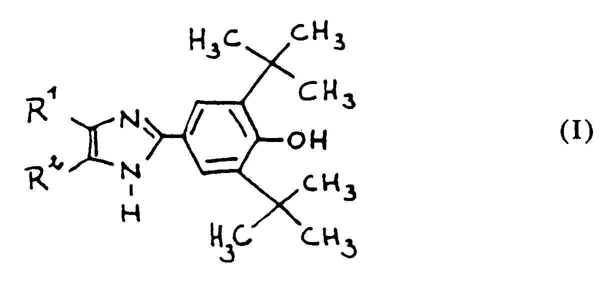 dans laquelle R¹ représente un groupe de formule générale II 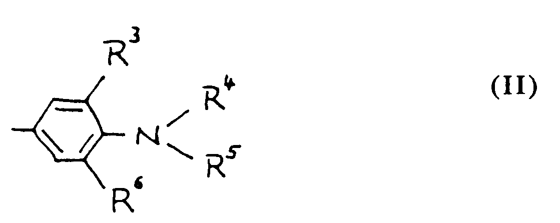 dans laquelle
R³ et R⁶ représentent un atome d'hydrogène et
R⁴ et R⁵, qui peuvent être identiques ou différents, représentent un groupe alkyle en C₁-C₆ ou
R³/R⁴ et/ou R⁵/R⁶ représentent chaque fois ensemble un groupe alkylène en C₃ ou C₄, et
R² représente un groupe alkyle en C₁-C₆,
ou un de ses sels formés avec un acide fort.

9. Agent selon la revendication 8, pour la détermination de créatine-kinase.

10. Agent selon l'une des revendications 8 ou 9, caractérisé en ce que les substances nécessaires pour la détermination de la substance contenue dans l'échantillon se trouvent sur un support solide.

11. Diarylimidazole de formule générale I 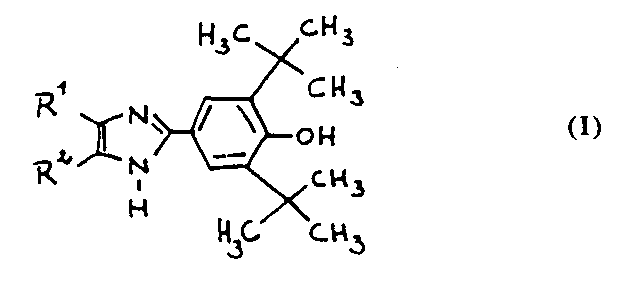 dans laquelle R¹ représente un groupe de formule générale II 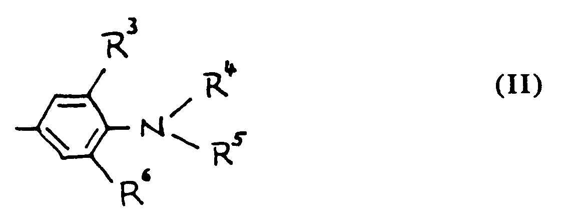 dans laquelle
R³ et R⁶ représentent un atome d'hydrogène et
R⁴ et R⁵, qui peuvent être identiques ou différents, représentent un groupe alkyle en C₁-C₆ ou
R³/R⁴ et/ou R⁵/R⁶ représentent chaque fois ensemble un groupe alkylène en C₃ ou C₄, et
R² représente un groupe alkyle en C₁-C₆,
ainsi que ses sels formés avec un acide fort,
excepté le composé qui est le chlorhydrate de 2-(3',5'-di-tert.butyl-4'-hydroxyphényl)-4(5)-4''-diméthylaminophényl)-5(4)-méthyl-(1H)-imidazole.

12. Diarylimidazole selon la revendication 1, caractérisé en ce que R¹ représente un groupe p-diéthylamino-phényle, un groupe p-di-n-propylamino-phényle ou un groupe julolidine, et R² représente un groupe méthyle.

13. 2-(3',5'-di-tert.-butyl-4'-hydroxyphényl)-4(5)-(9''-julolidyl)-5(4)-méthyl-(1H)-imidazole ou un de ses sels, selon la revendication 1.

14. Procédé de préparation d'un composé selon la revendication 1, caractérisé en ce que l'on condense une α-dicétone de formule III 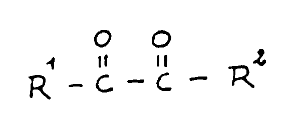 dans laquelle R¹ et R² ont la même signification que dans la revendication 1, avec un aldéhyde de formule IV 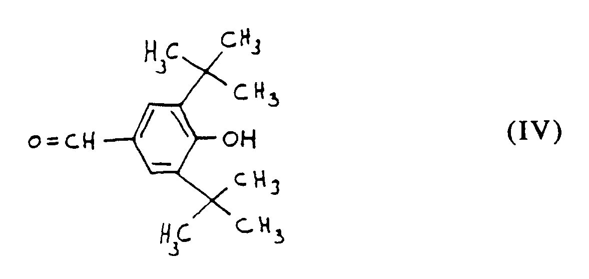 avec de l'ammoniac en solution acide, et ensuite, on effectue un traitement ultérieur éventuellement avec un acide.
